# EUROPÄISCHE PATENTSCHRIFT

(11) **EP 1 274 769 B1**
(45) Veröffentlichungstag und Bekanntmachung des Hinweises auf die Patenterteilung: **16.01.2013**
(21) Anmeldenummer: 01919451.3
(22) Anmeldetag: 12.04.2001
(51) Int. Cl.: C08G 73/02, A61K 6/10

(54) **Elastomermasse mit Basiskomponente und Katalysatorkomponente**
Elastomer composition with base and catalyst constituents
Masse élastomérique comprenant un composant de base et un catalyseur

(30) Priorität: 17.04.2000 DE 10018918
(43) Veröffentlichungstag der Anmeldung: 15.01.2003
(73) Patentinhaber: 3M Deutschland GmbH, 41453 Neuss (DE)
(72) Erfinder: ECKHARDT, Gunther, 82346 Frieding (DE); WANEK, Erich, 86916 Kaufering (DE); KUPPERMANN, Bernd, 82211 Herrsching (DE)
(74) Vertreter: Brem, Roland
(86) Internationale Anmeldenummer: PCT/EP2001/004217
(87) Internationale Veröffentlichungsnummer: WO 2001/079328

(56) Entgegenhaltungen:
- EP-A- 0 279 238
- DE-A- 19 753 461
- US-A- 3 453 242
- US-A- 3 842 019

## Beschreibung

Die Erfindung betrifft Zubereitungen auf der Grundlage von Aziridinopolyethern, die nach Mischung von zwei getrennt gelagerten Komponenten durch polymerbildende Reaktionen aushärten. Insbesondere beschreibt die Erfindung solche Zubereitungen, die sich durch eine gut einstellbare, relativ lange Verarbeitungszeit nach erfolgter Mischung sowie einen geringen Säuregrad der Katalysatorkomponente auszeichnen.

Für die Verwendung von zweikomponentigen Zubereitungen auf der Grundlage von Aziridinogruppen-haltigen Verbindungen ist es wichtig, den Verlauf der Umsetzungsgrad-/Zeitkurve entsprechend den jeweiligen Anforderungen einstellen zu können.

Den Verarbeiter interessieren an diesem Kurvenverlauf insbesondere zwei Kennwerte: die Verarbeitungszeit und die Zeit bis zur Erreichung der Weiterverarbeitbarkeit der aushärtenden Masse.

Als Verarbeitungszeit wird hierbei der Zeitraum zwischen der vollständigen Vermischung der zwei Komponenten und der beginnenden Aushärtung der gemischten Zubereitung bei Raumtemperatur verstanden. Dieser Polymerisationsbeginn wird als der Zeitpunkt angesehen, bei dem eine gemischte zweikomponentige Zubereitung aus der plastischen Phase in die elastische Phase übergeht und deutliche Veränderungen, wie Hautbildung, Fädenziehen und stark verringerte Fließfähigkeit zeigt.

Die gemischte Zubereitung ist bis kurz vor diesem Zeitpunkt noch verarbeitbar und zeigt das erforderliche Anfließen an die jeweiligen Substrate.

Üblicherweise wünscht der Verarbeiter eine Verarbeitungszeit von etwa 0,5 bis 4 Minuten bei Raumtemperatur, um genügend Zeit zu haben, die gegebenenfalls mehrfache Platzierung und Dosierung der aushärtenden Masse und Korrekturmaßnahmen ausrühren zu können. Hierbei erweist sich die Einstellung langer Verarbeitungszeiten als besonders schwierig.

Die Forderung nach langen Verarbeitungszeiten ist meist verbunden mit dem Wunsch, die Weiterverarbeitbarkeit der aushärtenden Masse nach einem möglichst kurzen Zeitraum möglich zu machen.

Für einen besonders wichtigen Anwendungsfall der dentalen Abformung mit zweikomponentigen Zubereitungen auf der Grundlage von Aziridinogruppen-haltigen Verbindungen bedeutet das:

Es sind Verarbeitungszeiten bei Raumtemperaturen von 2 bis 4 Minuten zu erreichen und die Weiterverarbeitbarkeit der Abformung in weniger als 7 Minuten nach Mischungsende zu gewährleisten. Als Weiterverarbeitung wird beispielsweise im Dentalbereich die Entnahme des ausgehärteten Abdrucks aus dem Mund des Patienten verstanden. Dies darf nicht zu früh geschehen, da ansonsten durch die noch nicht fertig abgebundene Masse die Präzision des Abdrucks zerstört wird.

Eine wichtige Forderung besteht weiterhin darin, dass die Rezepturänderungen, die die beschriebenen Kennzahlen des zeitlichen Abbindeverlaufs ermöglichen, keinen negativen Einfluss auf wichtige Eigenschaften der ausgehärteten elastischen Massen haben dürfen.

Die Einstellung des zeitlichen Abbindeverlaufs erfolgt bei zweikomponentigen Zubereitungen auf der Grundlage von Aziridinopolyethern durch das Initiierungssystem, d. h. durch Wechselwirkung von geeignet gewählten Startern und Verzögerern.

Es ist seit langem bekannt, dass N-Alkylaziridinoverbindungen unter Einwirkung von sauer wirkenden Verbindungen aushärten können (H. Bestian, Methoden der Organischen Chemie (Houben-Weyl), XII/1 (1958)).

Eine zusammenfassende Darstellung der für die Aushärtung von N-Alkylaziridinoverbindungen verwendeten Startersubstanzen ist in O. C. DERMER, G. E. HAM, "Ethylenimine and other Aziridines" Academic Press (1969) enthalten.

Als prinzipiell geeignete Starter haben sich demnach eine große Anzahl von Verbindungsklassen und Verbindungen erwiesen.

Allerdings wird die Auswahl der tatsächlich einsetzbaren Starter für zahnmedizinische und zahntechnische Abformmassen durch eine Reihe zusätzlicher Anforderungen stark eingeschränkt. So dürfen während der Aushärtung keine unangenehm riechenden Verbindungen entstehen. Weiterhin sollten bei der Herstellung, Abfüllung und Dosierung der Katalysatorkomponenten keine Korrosionserscheinungen an Metallteilen und produktberührenden Kunststoffteilen auftreten. Außerdem sollen selbst bei unsachgemäßer Lagerung sowie nicht bestimmungsgemäßer Verwendung der unvermischten Komponenten und insbesondere der Katalysatorkomponente keine Hautreizungen beim Praxispersonal oder beim Patienten auftreten.

Aus der DE-A-197 53 461 sind lagerstabile, kationisch polymerisierende Zubereitungen mit verbessertem Härtungsverhalten auf der Basis von N-Alkylaziridinogruppen-haltigen Verbindungen bekannt, die zur Erhöhung der Lagerstabilität lösliche und/oder feinteilige Erdalkali- und/oder Alkalimetallverbindungen enthalten. Diese können sowohl der Katalysatorkomponente als auch der Basiskomponente zugegeben werden, wobei die Zugabe zur Basiskomponente die bevorzugte Ausführungsform ist.

Des weiteren ist aus der DE-A-197 53 456 bekannt, daß man die Verarbeitungszeit von kationisch aushärtenden Zubereitungen auf der Basis von Aziridinopolyethern durch den Zusatz von Alkali- bzw. Erdalkaliverbindungen als die Abbindung verzögernde Zusätze wunschgemäß einstellen kann. Die Alkali- bzw. Erdalkaliverbindungen werden in gelöster als auch in feinteiliger, fester Form in die Basispaste eingebracht.

Der Nachteil der oben beschriebenen Zusammensetzungen besteht darin, daß die als Katalysator eingesetzte Säure die mit einer Aluminiumbeschichtung versehenen Beutel, in denen die Basis- und Katalysatorkomponente verpackt werden, angreift und zerstört. Diese Nachteile treten unabhängig davon auf, ob die Alkali- oder Erdalkaliverbindungen in der Basispaste vorhanden sind, wie es die DE-A-197 53 456 vorschreibt, oder ob sie gemäß der DE-A-197 53 461 in der Basis- oder in der Katalysatorpaste vorliegen.

EP 0 279 238 beschreibt Präparate für dentale Zwecke enthaltend drei räumlich voneinander getrennte Bestandteile, nämlich (a) mindestens eine Aziridinverbindung, (b) mindestens einen Sulfoniumsalz-Initiator für (a) und (c) mindestens eine lösbare oder dispergierbare, ionogene Verbindung, die ein Anion aufweist, das nucleophiler ist als das Anion von (b).

US 3,842,019 beschreibt Sulfonsäuresalze, die als latente Katalysatoren bei der Aushärtung oder der Polymerisation von kationisch polymerisierbaren Monomeren, wie Epoxide, Vinylether, N-Vinylverbindungen, Aziridine, ethylenisch ungesättigten Kohlenwasserstoffverbindungen und Acetalen verwendet werden können.

Aufgabe der Erfindung ist daher die Bereitstellung von Katalysatorkomponenten für zweikomponentige Zubereitungen auf der Grundlage von N-Alkylaziridinogruppen-haltigen Verbindungen, wobei diese Katalysatorkomponenten eine gut reproduzierbare Einstellung des Abbindeverlaufs und der resultierenden Eigenschaften gewährleisten sowie auch im ungemischten Zustand keine Reizung, Verätzung, Korrosion oder Zerstörung der Verpackung bewirken.

Die Aufgabe wird gelöst durch eine Elastomermasse wie sie in den Ansprüchen beschrieben ist, enthaltend eine Basiskomponente und eine Katalysatorkomponente, enthaltend:
(A) mindestens eine Brönstedsäure,
(B) Wasser,
(C) mindestens eine antacid wirkende Verbindung aus der Gruppe, bestehend aus den Oxiden, Hydroxiden, Carbonaten und/oder Carboxylaten der Elemente Aluminium, Chrom, Kupfer, Germanium, Mangan, Blei, Antimon, Zinn, Tellur, Titan und/oder Zink,
(D) gegebenenfalls mindestens ein inertes Verdünnungsmittel,
(E) gegebenenfalls mindestens einen Modifikator,
wobei die Mischung aus einem Gewichtsteil der Katalysatorkomponente mit drei Gewichtsteilen Bisaziridinopolyether mit einer mittleren Iminoäquivalentmasse von 3100, herstellbar aus einem Polyetherdiol, das Ethylenoxid- und Tetrahydrofuran-Einheiten im Molverhältnis 1 : 3,5 umfasst, innerhalb von 0,5 bis 20 Minuten zu einer festen Elastomer-Masse erstarrt, die nach 24 Stunden Lagerzeit bei Raumtemperatur eine Shore A-Härte von mindestens 20 gemäß DIN 53505 aufweist.

Die hier angegebenen Zeiten sind nicht als Verarbeitungszeit oder Zeitpunkt der Weiterverarbeitbarkeit im Sinne des vorher gesagten zu verstehen. Es handelt sich um die im Sinne eines Testsystems zu erreichenden Erstarrungszeiten.

Der als Testsubstanz für die Katalysatorkomponente verwendete Bisaziridinopolyether wird aus einem Polyetherdiol, das durch kationische Copolymerisation von Ethylenoxid und Tetrahydrofuran unter katalytischer Wirkung von Borfluorid-Etherat zugänglich ist, wobei durch die Reaktionsführung ein molares Einbauverhältnis von Ethylenoxid zu Tetrahydrofuran von 1 : 3,5 eingestellt wird, durch Umsetzung mit Crotonsäureanhydrid und nachfolgende Anlagerung von Ethylenimin, wie In US-3,453,242, Beispiel 13 dargelegt, synthetisiert.

Die Bestandteile der Katalysatorkomponenten sind wie folgt definiert:
(A) 0,1 bis 50 Gew.-%, bevorzugt 1 bis 20 Gew.-% mindestens einer Brönstedsäure,
(B) 0,1 bis 20 Gew.-%, bevorzugt 0,5 bis 10 Gew.-% Wasser,
(C) 0,1 bis 50 Gew.-%, bevorzugt 0,5 bis 10 Gew.-% mindestens einer der genannten antacid wirkenden Verbindungen,
(D) 0 bis 95 Gew.-%, bevorzugt 0 bis 70 Gew.-% mindestens eines inerten Verdünnungsmittels,
(E) 0 bis 50 Gew.-%, bevorzugt 0 bis 30 Gew.-% von Modifikatoren, einschließlich Füllstoffen, Farbstoffen, Pigmenten, Thixotropiemitteln, Fließverbesserern, polymeren Eindickern, oberflächenaktiven Substanzen, Geruchsstoffen und Geschmacksstoffen sowie die Polymerbildung verzögernden Verbindungen,
wobei die Gew.-%-Angaben jeweils auf das Gesamtgewicht der Katalysatorkomponente bezogen sind.

Es ist überraschend, dass durch den Einsatz von antacid wirkenden Verbindungen in Brönstedsäure-haltigen Katalysatorkomponenten trotz des sehr stark reduzierten Säuregrades eine ausreichend schnelle Aushärtung der angemischten Zubereitung erreicht werden kann, dass die Verarbeitungszeit dieser Zubereitungen im gewünschten Bereich über die Wasserkonzentration einstellbar ist und dass die erfindungsgemäße Katalysatorkomponente keine Reizungen oder Verätzungen hervorruft und auch die Verpackung (mit Metall beschichtete Beutel) nicht angreift.

Für den Einsatz als Säure gemäß Bestandteil (A) kommen eine große Anzahl von Verbindungsklassen und sauren Verbindungen in Betracht.

Prinzipiell sind sowohl organische als auch anorganische Säuren geeignet. Die Geschwindigkeit der Härtungsreaktion zeigt neben anderen Abhängigkeiten auch eine deutliche Abhängigkeit von der Säurestärke. So zeichnen sich sehr starke Säuren, wie Hexafluoroantimonsäure, Hexafluorophosphorsäure oder Tetrafluoroborsäure durch hohe Geschwindigkeiten der kationischen Polymerisation aus. Hohe Reaktionsgeschwindigkeiten werden auch von Sulfonsäuren, wie 4-Toluolsulfonsäure, 4-Phenolsulfonsäure, 4-Brombenzolsulfonsäure, 4-Chlorbenzolsulfonsäure, Benzolsulfonsäure, Alkylbenzolsulfonsäuren, insbesondere Dodecylbenzolsulfonsäure, Naphthalin-2-sulfonsäure und Alkansulfonsäuren erreicht. Die Verwendung von Phosphonsäuren, wie Vinylphosphonsäure und Propylphosphonsäure ist ebenfalls möglich.

Der Einsatz von polymeren Säuren, wie Polyvinylphosphonsäure, Polyacrylsäure, Copolymersäuren, hergestellt aus Maleinsäureanhydrid mit anderen Monomeren ist ebenso möglich, wenn es gelingt, einen lagerstabilen Verteilungszustand dieser Polymeren in der Katalysatorkomponente zu erreichen.

ist ebenso möglich, wenn es gelingt, einen lagerstabilen Verteilungszustand dieser Polymeren in der Katalysatorkomponente zu erreichen.

Weiterhin können auch gesättigte und ungesättigte Carbonsäuren, wie Propionsäure, Bernsteinsäure, Weinsäure, Trimellitsäure, Benzoesäure, Phenylessigsäure, Zitronensäure, Maleinsäure, Adipinsäure, o-Chlorbenzoesäure oder Umsetzungsprodukte von mehrwertigen Alkoholen und Säureanhydriden, wie Maleinsäureanhydrid und Bernsteinsäureanhydrid eingesetzt werden.

Die erfindungsgemäßen Zubereitungen enthalten als Bestandteil (A) mindestens eine Säure; die Verwendung von mehreren Säuren ist möglich und kann zur Einstellung des Abbindeverlaufs zweckmäßig sein.

Das gemäß Bestandteil (B) verwendete Wasser kann entweder den Säuren zugesetzt oder zu einem späteren Zeitpunkt der Herstellung der Katalysatorkomponente der Zubereitung zugegeben werden.

Bevorzugt ist die Verwendung wasserhaltiger Säuren als Bestandteil (A), wobei die Konzentrationseinstellung auf den gewünschten Wert im Zuge der Herstellung einer Vorlösung realisiert werden kann.

Die Wassermenge ist von der Löslichkeit der Säuren in den weiteren Bestandteilen der Katalysatorkomponente, dem gewünschten Aushärteverlauf und weiterer Eigenschaften der Katalysatorkomponente und der ausgehärteten Abformmasse abhängig.

Zur Erzielung einer besseren Löslichkeit der Brönstedsäure in der Katalysatorkomponente ist es jedoch zweckmäßig, den größeren Teil des notwendigen Wassers der Katalysatorkomponente zuzugeben.

Als antacid wirkende Verbindungen im Sinne der Erfindung entsprechend Bestandteil (C) werden die Oxide, Hydroxide, Carbonate und Carboxylate der Elemente Aluminium, Chrom, Kupfer, Germanium, Mangan, Blei, Antimon, Zinn, Tellur, Titan und Zink eingesetzt. Die Verwendung von Aluminium- und Zinkverbindungen ist bevorzugt.

Mit besonderem Vorteil werden Zinkverbindungen, wie Zinkhydroxid, Zinkoxid, Zinkcarbonat oder Gemische aus diesen Verbindungen verwendet.

Das Verhältnis zwischen den Bestandteilen (A) und (C) kann in weiten grenzen variiert werden. Es hat sich jedoch als vorteilhaft erwiesen, auf ein Säureäquivalent aus dem Bestandteil (A) 0,5 bis 2,0 Basenäquivalente aus dem Bestandteil (C) einzusetzen. Ein Verhältnis von einem Säureäquivalent zu 0,7 bis 1,2 Basenäquivalent ist besonders bevorzugt.

Als inertes Verdünnungsmittel entsprechend Bestandteil (D) können Polyetherpolyole, wie Polypropylenglykole oder Mischpolyetherole mit Tetrahydrofuran- und/oder Ethylenoxid- und/oder Propylenoxid-Einheiten, Polyesterpolyole, wie Polycaprolactondiole und Polycaprolactontriole, Polycarbonatdiole, aliphatische Ester, Öle, Fette, Wachse, aliphatische Kohlenwasserstoffe, araliphatische Kohlenwasserstoffe sowie ein- oder mehrfunktionelle Ester von mehrwertigen Säuren, wie Phthalsäure oder Zitronensäure oder Ester oder Amide von Alkylsulfonsäuren und Arylsulfonsäuren verwendet werden.

Der Katalysatorkomponente können als Bestandteil (E) Modifikatoren zugesetzt werden. Diese umfassen feinteilige Füllstoffe, wie Alumosilikate, Kieselsäuren, Quarzmehl, Wollastonit, Glimmermehl und Diatomeenerde sowie Farbstoffe und Pigmente, deren Zusatz eine bessere Beurteilung der Mischgüte ermöglicht und die Verwechslungsgefahr vermindert, Thixotropiemittel, wie feindisperse Kieselsäuren und andere das Fließverhalten beeinflussende Zusätze, wie polymere Eindicker, weiterhin oberflächenaktive Substanzen zur Einstellung des Anfließverhaltens sowie Geruchsstoffe und/oder Geschmacksstoffe.

Die notwendige Wasserkonzentration in der aushärtenden Zubereitung kann durch Versuche ermittelt werden, wobei zumindest ein Teil des notwendigen Wassers vor der Mischung auch in der Basiskomponente vorliegen kann.

Die geeigneten Konzentrationen an Wasser und antacid wirkenden Verbindungen sind über einen einfachen Test bestimmbar.

Die Basiskomponente kann nur aus Aziridinomonomeren bestehen, wie sie beispielsweise als Testsubstanz für die Katalysatorkomponente beschrieben sind.

Zur Einstellung eines verbesserten Handlings und zur Erzielung der gewünschten Eigenschaftskombination der Elastomer-Masse enthält die Basiskomponente:
(I) 5 bis 100 Gew.-% von mindestens einer N-Alkylaziridinoverbindung mit Aziridinoäquivalentmassen von 500 bis 25.000 g/Äquivalent,
(II) 0 bis 95 Gew.-% mindestens eines inerten Verdünnungsmittels,
(III) 0 bis 80 Gew.-% von Modifikatoren.

Die beiden Komponenten werden getrennt gelagert und zur Verarbeitung in einem Verhältnis von Katalysatorkomponente zu Basiskomponente von 5 : 1 bis 1 : 20, vorzugsweise von 1 : 1 bis 1 : 10 miteinander vermischt.

Die Herstellung der erfindungsgemäßen Katalysatorkomponenten kann in unterschiedlicher Weise erfolgen.

So kann die Brönstedsäure zusammen mit Wasser bei 20° C bis 90° C, bevorzugt 50° C bis 70° C in einem Teil oder der Gesamtmenge des inerten Verdünnungsmittels gemäß Bestandteil (D) gelöst werden und vor oder nach der Einarbeitung von Füll- und Farbstoffen die antacid wirkende Verbindung als Pulver, als Paste oder als Suspension zugesetzt werden.

Nach einer bevorzugten Ausführungsform der Katalysatorkomponentenherstellung wird aus den Bestandteilen (A), (B), (D) und (E) eine Paste hergestellt und danach die antacid wirkenden Verbindungen (C) zugegeben, wobei es besonders bevorzugt ist, die antacid wirkenden Verbindungen in Form einer Paste oder einer Suspension, die jeweils aus einem Teil des Verdünnungsmittels und den antacid wirkenden Verbindungen hergestellt wurde, zu verwenden.

Die erfindungsgemäßen Katalysatorkomponenten für zweikomponentige durch polymerbildende Reaktionen aushärtende Zubereitungen auf der Basis von N-Alkylaziridinoverbindungen können in Abhängigkeit von der Zusammensetzung der Katalysatorkomponente und der Basiskomponente für das Verkleben von Substraten, für das Abdichten, das Beschichten und das Vergießen eingesetzt werden.

Dabei kann die Dosierung der beiden Komponenten nach Sicht (Stranglängenvergleich), nach Gewicht, über vordosierte Packungseinheiten und nachfolgende Handanmischung, aus Doppelkammerkartuschen mit statischem Mischrohr oder mittels Volumendosieranlagen mit nachgeschaltetem statischem oder dynamischem Mischer erfolgen.

Zur Erzielung optimaler Ergebnisse ist eine hohe Mischgüte erforderlich. Dagegen ist die Toleranz des Mischungsverhältnisses im allgemeinen relativ groß und kann beispielsweise bei einem vorgegebenen Verhältnis von Katalysatorkomponente zu Basiskomponente von 1 : 5 den Bereich 0,8 bis 1,2 : 5 umfassen, ohne dass einsatzbeschränkende Eigenschaftsänderungen feststellbar sind.

Die erfindungsgemäßen Zubereitungen können mit Vorteil zur Abformung von Gegenständen oder Körperteilen eingesetzt werden, wobei mit den Zubereitungen gemäß der Erfindung auf Grund ihres hervorragenden Anfließverhaltens detailgetreue Abformungen erhalten werden.

Mit besonderem Vorteil werden die Zubereitungen gemäß der Erfindung bei der zahnmedizinischen und zahntechnischen Abformung eingesetzt

Bei der zahnmedizinischen Abformung erweist sich das gute Anfließverhalten an den feuchten Zahn und das feuchte Zahnfleisch sowie die Unempfindlichkeit der Präzision der Abformung gegenüber Speichel und Blut als großer Vortell.

Die aus den erfindungsgemäßen Zubereitungen hergestellten Massen, insbesondere Dentalmassen sind üblicherweise in Behältnissen und Mischvorrichtungen, enthalten, wie Kartuschen, Beutel, Abformlöffel, statische und dynamische Mischer bzw. Mischgeräte.

Die Erfindung wird durch die nachfolgenden Beispiele näher beschrieben.

### Beispiele

Mit Hilfe von Laborknetem wurden die in Tabelle 1 beschriebenen Katalysatorkomponenten im 100 g-Maßstab hergestellt.

Hierbei wurden die Säuren (A) in Wasser (B) gelöst, 70 % des angegebenen Verdünnungsmittels (D) zugegeben und der Füllstoff (E) eingeknetet und dann die Suspension der antacid wirkenden Verbindungen (C) in 30 % der angegebenen Verdünnungsmittelmenge (D) hinzugefügt.

Die Herstellung der Basiskomponente, deren Zusammensetzung in Tabelle 3 angegeben ist, erfolgte im 500 g-Maßstab.

In Tabelle 4 sind die Mischungen zusammengestellt, die unter Verwendung der in Tabelle 1 beschriebenen Katalysatorkomponenten und der in Tabelle 3 beschriebenen Basiskomponente im jeweils angegebenen Gewichtsverhältnis untersucht wurden. Die Mischungen wurden durch Anspateln auf dem Mischblock innerhalb von 30 Sekunden zubereitet und zur Bestimmung der in Tabelle 4 zusammengestellten Eigenschaften eingesetzt.

Die Mundentnahmezeit konnte als Mittelwert von jeweils drei Abdrucknahmen an drei verschiedenen Probanden in Form eines vollständigen Oberkiefer-Abdruckes ermittelt werden.

Die Herstellung der Bisaziridinopolyether aus Polyetherdiolen, die durch Copolymerisation von Ethylenoxid und Tetrahydrofuran synthetisiert werden, ist prinzipiell in Beispiel 13 der US-3,453,242 beschrieben.

Der als Testsubstanz für die Katalysatorkomponente verwendete Bisaziridinopolyether wird aus einem Polyetherdiol, das durch kationische Copolymerisation von Ethylenoxid und Tetrahydrofuran unter katalytischer Wirkung von Borfluorid-Etherat zugänglich ist, wobei durch die Reaktionsführung ein molares Einbauverhältnis von Ethylenoxid zu Tetrahydrofuran von 1 : 3,5 eingestellt wird, durch Umsetzung mit Crotonsäureanhydrid und nachfolgende Anlagerung von Ethylenimin, wie in US-3,453,242, Beispiel 13 dargelegt, synthetisiert.

**Tabelle 1**

| Zusammensetzung der erfindungsgemäßen Katalysatorkomponenten | | | | | | | | | | | | |
|---|---|---|---|---|---|---|---|---|---|---|---|---|
| | Bestandteil | | Gew.-% | | | | | | | | | |
| | | | K1 | K2 | K3 | K4 | K5 | K6 | K7 | K8 | K9 | K10 |
| (A) | - | p-Toluolsulfonsäure-Monohydrat | 10,1 | 9,9 | 10,0 | 6,7 | 4,1 | 6,0 | 8,9 | 9,8 | 8,3 | 7,3 |
| | - | Tetrafluoroborsäure | — | — | — | 1,2 | 2,7 | — | — | — | — | — |
| | - | C₁₅-n-Alkansulfonsäure | — | — | — | — | — | 3,2 | 1,0 | — | — | 0,2 |
| (B) | - | Destilliertes Wasser | 5,5 | 4,7 | 3,7 | 5,9 | 5,0 | 5,5 | 6,1 | 4,3 | 3,9 | 5,7 |
| (C) | - | Zinkoxid | 2,0 | 2,0 | 2,0 | 1,9 | 1,5 | 1,6 | 1,9 | - | 1,5 | 1,5 |
| | - | Zinkcarbonat | — | — | — | — | 0,5 | — | — | — | — | — |
| | - | Aluminiumhydroxid | — | — | — | — | — | — | — | 1,3 | 0,2 | — |
| (D) | - | Polypropylenglykol mit der Molmasse von 2100 g/Mol | — | — | — | 31,0 | — | 29,3 | 46,0 | — | 20,7 | 10,3 |
| | - | Poly(-ethylen, -propylen)glykol mit einer Molmasse von 3400 g/Mol | 60,9 | 62,1 | 63,6 | 29,0 | 65,5 | 33,3 | 16,0 | 58,9 | 43,2 | 50,4 |
| (E) | - | Diatomeenerde | — | 5,3 | — | — | — | 10,8 | — | 12,7 | 1,4 | 18,3 |
| | - | Fällungskieselsäure, hydrophob, mittlere Teilchengröße: 10 µm | 21,5 | 16,0 | 20,7 | 24,3 | 20,7 | 10,3 | 20,1 | 13,0 | 20,8 | 6,3 |

### Eignungstest:

Je ein Gewichtsteil der in Tabelle 1 beschriebenen Katalysatorkomponenten K1 bis K10 wurden mit drei Gewichtsteilen der Testsubstanz: Bis-aziridinopolyether mit einer Iminoäquivalentmasse von 3100 g/Äquivalent, hergestellt aus einem Polyetherdiol, das aus Ethylenoxid- und Tetrahydrofuran-Einheiten im Molverhältnis 1 : 3,5 besteht, vermischt.

Die Mischungen erstarrten bei 23° C in einem Zeitraum von drei bis fünf Minuten zu elastischen Massen, die nach einer Lagerzeit von 24 Stunden bei Raumtemperatur eine nach DIN 53505 gemessene Shore A-Härte von 50 bis 70 aufwiesen.

Alle erfindungsgemäßen Katalysatorkomponenten hatten einen geringen Säuregrad entsprechend den in Tabelle 2 angegebenen pH-Werten, die mit einer üblichen Elektrode gemessen wurden, aus der während der Messung als Elektrolyt eine KCI-Lösung austrat.

Die erfindungsgemäßen Katalysatorkomponenten bewirkten keine Hautreizung. Eine Zerstörung von Verpackungskunststoffen, wie Polyamid, Polyoxymethylendiazetat oder Laminatfolie trat auch nach 3-monatiger Lagerung bei 50° C im Kontakt mit den erfindungsgemäßen Katalysatorkomponenten nicht ein.

**Tabelle 2 Säuregrade der erfindungsgemäßen Katalysatorkomponenten, ermittelt als pH-Wert**

| Katalysatorkomponente gemäß Tabelle 1 | pH-Wert |
|---|---|
| K1 | 4,6 |
| K2 | 4,4 |
| K3 | 4,2 |
| K4 | 3,6 |
| K5 | 3,2 |
| K6 | 4,1 |
| K7 | 4,5 |
| K8 | 2,2 |
| K9 | 3,7 |
| K10 | 4,6 |

### Vergleichsbeispiel

Es wurde eine Katalysatorkomponente folgender Zusammensetzung hergestellt.

| **Bestandteil** | | **Gew.-%** |
|---|---|---|
| - | p-Toluolsulfonsäure-Monohydrat | 9,9 |
| - | Destilliertes Wasser | 4,0 |
| - | Fällungskieselsäure, hydrophob mittlere Teilchengröße: 10 µm | 20,8 |
| - | Poly(-ethylen, -propylen)glykol mit einer Molmasse von 3400 g/Mol | 65,3 |

Die Katalysatorkomponente hatte einen sehr hohen Säuregrad (pH-Wert von 0), zeigte eine ätzende Wirkung auf der feuchten Haut und zerstörte Verpackungskunststoffe bei längerer Lagerung.

Außerdem konnten mit dieser Katalysatorkomponente keine Verarbeitungszeiten über 100 Sekunden erreicht werden, was jedoch für eine Reihe von Anwendungen notwendig ist.

**Tabelle 3**

| **Zusammensetzung der Basiskomponente** | |
|---|---|
| Bestandteil | Gew.-% |
| Gemisch aus Bisaziridinopolyethern mit einer mittleren Iminoäquivalentmasse von 3190, hergestellt aus einem Polyetherdiol, das aus Ethylenoxid- und Tetrahydrofuran-Einheiten im Molverhältnis 1 : 3,3 besteht, mit einem Gehalt an cyclischen oligomeren Polyethern von 0,2 %. | 55,1 |
| Dibenzyltoluol (Jarytherm DBT, Elf Atochem) | 20,0 |
| Hydrierter Rindertalg (Ewanol HY2, Unichema) | 13,2 |
| Diatomeenerde (Celatom MW 25, Chemag AG) | 11,7 |

**Tabelle 4**

| **Charakterisierung der hergestellten Mischungen der Katalysatorkomponenten mit der Basiskomponente gemäß Tabelle 2** | | | | | | | | | | |
|---|---|---|---|---|---|---|---|---|---|---|
| | Beispiele-Nr. | | | | | | | | | |
| | 1 | 2 | 3 | 4 | 5 | 6 | 7 | 8 | 9 | 10 |
| Verwendete Katalysatorkomponente (gemäß Tabelle 1) | K1 | K2 | K3 | K4 | K5 | K6 | K7 | K8 | K9 | K10 |
| Gewichtsverhältnis: Katalysatorkomponente zu Basiskomponente | 1 : 5,2 | 1 : 4,9 | 1 : 5,0 | 1 : 6,0 | 1 : 5,1 | 1 : 5,0 | 1 : 4,5 | 1 : 5,0 | 1 : 4,0 | 1 : 5,3 |

**Tabelle 5**

| **Verarbeitungsverhalten und erreichte Shore-A-Härten der Zubereitungen gemäß den Beispielen 1 bis 10 (Tabelle 4)** | | | | | | | | | | |
|---|---|---|---|---|---|---|---|---|---|---|
| Eigenschaft | Beispiele-Nr. | | | | | | | | | |
| | 1 | 2 | 3 | 4 | 5 | 6 | 7 | 8 | 9 | 10 |
| Verarbeitungszeit (23° C) Sekunden | 160 | 175 | 190 | 140 | 120 | 170 | 150 | 130 | 170 | 175 |
| Mundentnahmezeit Sekunden | 270 | 285 | 330 | 300 | 315 | 280 | 260 | 240 | 300 | 290 |
| Shore-A-Härte nach 24 Stunden gemäß DIN 53505 | 62 | 63 | 59 | 60 | 58 | 59 | 64 | 65 | 61 | 63 |

Aus Tabelle 1 in Verbindung mit den Tabellen 4 und 5 ist ersichtlich, dass durch die Auswahl der antaciden Verbindung, das molare Verhältnis der Säuren zu den antaciden Verbindungen und durch den Wassergehalt die gewünschte Verarbeitungszeit eingestellt werden kann. Dabei wird auch die Mundentnahmezeit verändert, liegt aber bei Verwendung der erfindungsgemäßen Katalysatorkomponenten in dem für Präzisionsabformungen üblichen Bereich.

Alle Mischungen der erfindungsgemäßen Beispiele 1 bis 10 (Tabelle 4) erfüllten die Anforderungen an eine elastische Abformmasse hinsichtlich Reißfestigkeit, Reißdehnung, bleibende elastische Deformation nach DIN/EN 24823 und führten zu Formkörpern, die nach einer Lagerzeit bei Raumtemperatur von 24 Stunden eine Shore A-Härte (Tabelle 5) deutlich über 20 ergaben.

Alle Mischungen der erfindungsgemäßen Beispiele 1 bis 10 (Tabelle 4) ergaben Abdrücke, die sich nach der Entnahme aus dem Mund nicht klebrig anfühlten und die sich durch eine augenscheinlich bestimmte sehr gute Zeichnungsschärfe auszeichneten.

Die Einzelkomponenten und auch die Mischungen zeigten keinen unangenehmen oder auffälligen Geruch.

## Patentansprüche

1. Elastomer-Masse enthaltend eine Basiskomponente, enthaltend:
(I) 5 bis 100 Gew.-% von mindestens einer N-Alkylaziridinoverbindung mit Aziridinoäquivalentmassen von 500 bis 25.000 g/Äquivalent,
(II) 0 bis 95 Gew.-% mindestens eines inerten Verdünnungsmittels,
(III) 0 bis 80 Gew.-% von Modifikatoren
und eine
Katalysatorkomponente enthaltend:
(A) 0,1 bis 50 Gew.-% mindestens einer Brönstedsäure,
(B) 0,1 bis 20 Gew.-% Wasser,
(C) 0,1 bis 50 Gew.-% mindestens einer antacid wirkenden Verbindung,
(D) 0 bis 95 Ges.-% mindestens eines inerten Verdünnungsmittels,
(E) 0 bis 50 Gew.-% von Modifikatoren,
wobei die Gew.-%-Angaben jeweils auf das Gesamtgewicht entweder der Katalysatorkomponente oder der Basiskomponentebezogen sind.

2. Elastomer-Masse nach Anspruch 1, wobei die mindestens eine antacid wirkende Verbindung gewählt ist aus der Gruppe, bestehend aus den Oxiden, Hydroxiden, Carbonaten und/oder Carboxylaten der Elemente Aluminium, Chrom, Kupfer, Germanium, Mangan, Blei, Antimon, Zinn, Tellur, Titan und/oder Zink.

3. Elastomer-Masse nach einem der vorangehenden Ansprüche, wobei pro Säureäquivalent der Brönstedsäure 0,5 bis 2,0 Basenäquivalente der antacid wirkenden Verbindung eingesetzt werden.

4. Elastomer-Masse nach einem der vorangehenden Ansprüche, wobei die antacid wirkende Verbindung Zinkoxid umfasst.

5. Elastomer-Masse nach einem der vorstehenden Ansprüche, wobei als Säure gemäß Bestandteil (A) Hexafluoroantimonsäure, Hexafluorophosphorsäure. Tetrafluoroborsäure, Sulfonsäuren, Phosphonsäuren, Polyvinylphosphonsäure, Polyacrylsäure oder Copolymersäuren, hergestellt aus Maleinsäureanhydrid mit anderen Monomeren, eingesetzt werden.

6. Elastomer-Masse nach einem der vorangehenden Ansprüche, wobei Bestandteil (A) gewählt ist aus: Arylsulfonsäuren, Alkylsulfonsäuren und Alkylbenzolsulfonsäuren.

7. Elastomer-Masse nach einem der vorangehenden Ansprüche, wobei Bestandteil (A) p-Toluolsulfonsäure oder Dodecylbenzolsulfonsäure umfasst.

8. Elastomer-Masse nach einem der Ansprüche 4 oder 6, wobei Zinkoxid und p-Toluolsulfonsäure im Molverhältnis 1 : 1,5 bis 1 : 2,5 eingesetzt werden.

9. Elastomer-Masse nach einem der vorstehenden Ansprüche, wobei als inertes Verdünnungsmittel entsprechend Bestandteil (D) Polyetherpolyole, Polyesterpolyole, Polycarbonatdiole, aliphatische Ester, Öle, Fette, Wachse, aliphatische Kohlenwasserstoffe, araliphatische Kohlenwasserstoffe sowie ein- oder mehrtunktionelle Ester von mehrwertigen Säuren oder Ester oder Amide von Alkylsulfonsäuren und Arylsulfonsäuren verwendet werden.

10. Verfahren zur Herstellung einer Elastomer-Masse nach einem der vorangehenden Ansprüche, wobei die Brönstedsäure zusammen mit Wasser bei 20° C bis 70° C in einem Teil des inerten Verdünnungsmittels gemäß Bestandteil (D) gelöst wird und zu der Lösung eine Suspension der antacld wirkenden Verbindungen zugegeben wird.

11. Verfahren zur Herstellung einer Elastomer-Masse nach einem der Ansprüche 1 bis 7, wobei aus den Verbindungen gemäß den Bestandteilen (A), (B), (D) und (E) eine Paste hergestellt wird und danach die antacid wirkenden Verbindungen zugegeben werden.

12. Verfahren nach Anspruch 8, wobei die Zugabe der antacid wirkenden Verbindungen in Form einer Paste oder einer Suspension erfolgt, die aus der antacid wirkenden Verbindung und einem Teil des inerten Verdünnungsmittels hergestellt wurde.

13. Verwendung der Katalysatorkomponente wie in einem der Ansprüche 1 bis 7 beschrieben zusammen mit einer eine N-Akylaziridinogruppen-haltige Verbindung umfassenden Basiskomponente zur Herstellung von härtbaren Zubereitungen.

14. Verwendung nach Anspruch 13, wobei das Gewichtsverhältnis zwischen der als Bestandteil (A) der Katalysatorkomponente verwendeten Brönstedsäure und der N-Alkylaziridinogruppen-haltigen Verbindung der Basiskomponente so gewählt wird, dass ein Verhältnis von Säureäquivalent der Brönstedsäure zu Iminäquivalent der N-Alkylaziridinogruppen-haltlgen Verbindung von 1 : 0,5 bis 1 : 5, bevorzugt 1 : 1 bis 1 : 3 und besonders bevorzugt von 1 : 1,2 bis 1 : 2,0 eingehalten wird.

15. Verwendung der Elastomer-Masse nach einem der Ansprüche 13 oder 14, wobei es sich bei der härtbaren Zubereitung um eine zahnmedizinische oder zahntechnische Abformung handelt.

## Claims

1. Elastomer composition comprising a base constituent, comprising:
(I) from 5 to 100% by weight of at least one N-alkylaziridino compound with aziridino equivalent weights of from 500 to 25 000 g/equivalent,
(II) from 0 to 95% by weight of at least one inert diluent,
(III) from 0 to 80% by weight of modifiers,
and a
catalyst constituent comprising:
(A) from 0.1 to 50% by weight of at least one Brønsted acid,
(B) from 0.1 to 20% by weight of water,
(C) from 0.1 to 50% by weight of at least one compound having an antacid effect,
(D) from 0 to 95% by weight of at least one inert diluent,
(E) from 0 to 50% by weight of modifiers,
the % by weight specifications being in each case based on the total weight either of the catalyst constituent or of the base constituent.

2. Elastomer composition according to Claim 1, in which the at least one compound having an antacid effect is chosen from the group consisting of oxides, hydroxides, carbonates and/or carboxylates of the elements aluminium, chromium, copper, germanium, manganese, lead, antimony, tin, tellurium, titanium and/or zinc.

3. Elastomer composition according to either of the preceding claims, in which use is made, per acid equivalent of the Brønsted acid, of from 0.5 to 2.0 base equivalents of the compound having an antacid effect.

4. Elastomer composition according to one of the preceding claims, in which the compound having an antacid effect comprises zinc oxide.

5. Elastomer composition according to one of the preceding claims, in which use is made, as acid according to component (A), of hexafluoroantimonic acid, hexafluorophosphoric acid, tetrafluoroboric acid, sulphonic acids, phosphonic acids, polyvinylphosphonic acid, polyacrylic acid or copolymer acids prepared from maleic anhydride with other monomers.

6. Elastomer composition according to one of the preceding claims, in which component (A) is chosen from: arylsulphonic acids, alkylsulphonic acids and alkylbenzenesulphonic acids.

7. Elastomer composition according to one of the preceding claims, in which component (A) comprises p-toluenesulphonic acid or dodecylbenzenesulphonic acid.

8. Elastomer composition according to either of Claims 4 and 6, in which zinc oxide and p-toluenesulphonic acid are used in the molar ratio from 1:1.5 to 1:2.5.

9. Elastomer composition according to one of the preceding claims, in which use is made, as inert diluents corresponding to component (D), of polyether polyols, polyester polyols, polycarbonate diols, aliphatic esters, oils, fats, waxes, aliphatic hydrocarbons, araliphatic hydrocarbons as well as mono- or polyfunctional esters of polyvalent acids or esters or amides of alkylsulphonic acids and arylsulphonic acids.

10. Process for the preparation of an elastomer composition according to one of the preceding claims, in which the Brønsted acid together with water is dissolved at 20°C to 70°C in a portion of the inert diluent according to component (D) and a suspension of the compounds having an antacid effect is added to the solution.

11. Process for the preparation of an elastomer composition according to one of Claims 1 to 7, in which a paste is prepared from the compounds according to the components (A), (B), (D) and (E) and then the compounds having an antacid effect are added.

12. Process according to Claim 11, in which the compounds having an antacid effect are added in the form of a paste or of a suspension which was prepared from the compound having an antacid effect and a portion of the inert diluent.

13. Use of the catalyst constituent as described in one of Claims 1 to 7, together with a base constituent comprising a compound comprising N-alkylaziridino groups, for the preparation of curable formulations.

14. Use according to Claim 13, in which the weight ratio of the Brønsted acid used as component (A) of the catalyst constituent to the compound comprising N-alkylaziridino groups of the base constituent is chosen so that a ratio of acid equivalent of the Brønsted acid to imine equivalent of the compound comprising N-alkylaziridino groups of from 1:0.5 to 1:5, preferably from 1:1 to 1:3 and particularly preferably from 1:1.2 to 1:2.0 is observed.

15. Use of the elastomer composition according to either of Claims 13 and 14, in which the curable formulation is a dental or prosthetic dental impression.

## Revendications

1. Masse d'élastomère contenant un composant de base, contenant :
(I) 5 à 100% en poids d'au moins un composé N-alkylaziridino présentant des masses d'équivalent aziridino de 500 à 25 000 g/équivalent,
(II) 0 à 95% en poids d'au moins un diluant inerte,
(III) 0 à 80% en poids de modificateurs
et un composant catalytique, contenant :
(A) 0,1 à 50% en poids d'au moins un acide de Brönsted,
(B) 0,1 à 20% en poids d'eau,
(C) 0,1 à 50% en poids d'au moins un composé à effet antiacide,
(D) 0 à 95% en poids d'au moins un diluant inerte,
(E) 0 à 50% en poids de modificateurs,
les indications en % en poids se rapportant à chaque fois au poids total, soit du composant catalytique, soit du composant de base.

2. Masse d'élastomère selon la revendication 1, ledit au moins un composé à effet antiacide étant choisi dans le groupe constitué par les oxydes, les hydroxydes, les carbonates et/ou les carboxylates des éléments aluminium, chrome, cuivre, germanium, manganèse, plomb, antimoine, étain, tellure, titane et/ou zinc.

3. Masse d'élastomère selon l'une quelconque des revendications précédentes, en utilisant, par équivalent acide de l'acide de Brönsted, 0,5 à 2,0 équivalents basiques du composé à effet antiacide.

4. Masse d'élastomère selon l'une quelconque des revendications précédentes, le composé à effet antiacide comprenant de l'oxyde de zinc.

5. Masse d'élastomère selon l'une quelconque des revendications précédentes, en utilisant comme acide selon le constituant (A) l'acide hexafluoroantimonique, l'acide hexafluorophosphorique, l'acide tétrafluoroborique, les acides sulfoniques, les acides phosphoniques, l'acide polyvinylphosphonique, l'acide polyacrylique ou les acides copolymères, préparés à partir d'anhydride de l'acide maléique avec d'autres monomères.

6. Masse d'élastomère selon l'une quelconque des revendications précédentes, le constituant (A) étant choisi parmi : les acides arylsulfoniques, les acides alkylsulfoniques et les acides alkylbenzènesulfoniques.

7. Masse d'élastomère selon l'une quelconque des revendications précédentes, le constituant (A) comprenant l'acide p-toluènesulfonique ou l'acide dodécylbenzènesulfonique.

8. Masse d'élastomère selon l'une quelconque des revendications 4 ou 6, l'oxyde de zinc et l'acide p-toluènesulfonique étant utilisés dans un rapport molaire de 1:1,5 à 1:2,5.

9. Masse d'élastomère selon l'une quelconque des revendications précédentes, en utilisant comme diluant inerte selon le constituant (D) des polyétherpolyols, des polyesterpolyols, des polycarbonatediols, des esters aliphatiques, des huiles, des graisses, des cires, des hydrocarbures aliphatiques, des hydrocarbures araliphatiques ainsi que des esters monofonctionnels ou polyfonctionnels d'acides polyvalents ou des esters ou des amides d'acides alkylsulfoniques et d'acides arylsulfoniques.

10. Procédé pour la préparation d'une masse d'élastomère selon l'une quelconque des revendications précédentes, l'acide de Brönsted étant dissous ensemble avec de l'eau à 20°C jusqu'à 70°C dans une partie du diluant inerte selon le constituant (D) et en ajoutant à la solution une suspension des composés à effet antiacide.

11. Procédé pour la préparation d'une masse d'élastomère selon l'une quelconque des revendications 1 à 7, en préparant à partir des composés selon les constituants (A), (B), (D) et (E) une pâte, puis en ajoutant les composés à effet antiacide.

12. Procédé selon la revendication 11, l'addition des composés à effet antiacide ayant lieu sous forme d'une pâte ou d'une suspension, qui a été préparée à partir du composé à effet antiacide et d'une partie du diluant inerte.

13. Utilisation du composant catalytique comme décrit dans l'une quelconque des revendications 1 à 7 ensemble avec un composant de base comprenant un composé contenant des groupes N-alkylaziridino pour la préparation de compositions durcissables.

14. Utilisation selon la revendication 13, le rapport pondéral entre l'acide de Brönsted utilisé comme constituant (A) du composant catalytique et le composé contenant des groupes N-alkylaziridino du composant de base étant choisi de manière telle qu'un rapport d'équivalent acide de l'acide de Brönsted à équivalent imino du composé contenant des groupes N-alkylaziridino de 1:0,5 à 1:5, de préférence 1:1 à 1:3 et de manière particulièrement préférée de 1:1,2 à 1:2,0 est respecté.

15. Utilisation de la masse d'élastomère selon l'une quelconque des revendications 13 ou 14, où il s'agit, pour la composition durcissable, d'un moulage de médecine ou de technique dentaire.
